# EUROPEAN PATENT APPLICATION

(11) **EP 4 781 952 A1**
(43) Date of publication of application: **29.07.2026**
(21) Application number: 25194694.3
(22) Date of filing: 07.08.2025
(51) Int. Cl.: A61F 5/01

(54) **HINGE FOR AN ORTHOSIS**

(30) Priority: 23.01.2025 TW 114102988
(71) Applicant: Oppo Medical Inc., Seattle Washington 98101 (US); Plus Meditech Co., Ltd, 70266 Tainan City (TW)
(72) Inventor: CHIANG, Yueh-Hua, 10690 Taipei City (TW); LEE, Kuo-Wei, 10690 Taipei City (TW); WU, Ming-Jhih, 10690 Taipei City (TW); SCHILLER, Rene Winfried, 70266 Tainan City (TW); WAGNER, Helmut, 70266 Tainan City (TW)
(74) Representative: Marks & Clerk LLP

(57) **Abstract**

A hinge includes a housing (1) having a retaining slot (122), a lower strut (2) and an upper strut (3) hinged with the housing and connected with each other to be synchronously rotated in opposite rotational directions, and an angularly restraining member (5, 5') including an angularly restraining body (51) which abuts against the lower and upper struts to define turnable angles thereof, and a retaining block (53) which is connected with an opposite side of the resilient arm (52) opposite to the angularly restraining body which is removably engaged in the retaining slot. The retaining block is operable to bring the resilient arm in resilient deformation to permit removal from the retaining slot.

## Description

The disclosure relates to an orthotic device, and more particularly to a hinge of an orthosis adapted to set the turning range of the orthosis.

A conventional knee orthosis generally includes a hinge for supporting the knees of a user and for adjusting the range of flexion and extension of the knees so as to avoid an injury to the knees due to excessive flexion and extension.

However, a conventional hinge for an orthosis has many components, involves a complicated construction at a high cost, and is not easy to operate to adjust the angle of the hinge.

Therefore, an object of the disclosure is to provide a hinge that can alleviate at least one of the drawbacks of the prior art.

According to an aspect of the disclosure, there is provided a hinge according to claim 1.

Other features and advantages of the disclosure will become apparent in the following detailed description of the embodiment with reference to the accompanying drawings. It is noted that various features may not be drawn to scale.
FIG. 1 is a schematic side view illustrating an embodiment of a hinge according to the disclosure.
FIG. 2 is a perspective view of the embodiment.
FIG. 3 is a fragmentary, exploded perspective view of the embodiment.
FIG. 4 is a fragmentary, exploded perspective view of the embodiment taken from another angle.
FIG. 5 is a sectional view taken along line V-V of FIG. 2.
FIG. 6 is a sectional view taken along line VI-VI of FIG. 2, illustrating a state in which a retaining block of an angularly restraining member is retained to a housing.
FIG. 7 is a sectional view taken along line VII-VII of FIG. 2.
FIG. 8 is a sectional view of the embodiment, illustrating a state in which the angularly restraining member is being mounted on the housing.
FIG. 9 is a fragmentary side view of the embodiment.
FIG. 10 is a fragmentary side view of the embodiment.
FIG. 11 is a sectional view similar to FIG. 6, illustrating a state in which the angularly restraining member is being removed from the housing.
FIG. 12 is a fragmentary side view of the embodiment, illustrating a state in which another angularly restraining member is being mounted on the housing.

It should be noted herein that for clarity of description, spatially relative terms such as "top," "bottom," "upper," "lower," "on," "above," "over," "downwardly," "upwardly" and the like may be used throughout the disclosure while making reference to the features as illustrated in the drawings. The features may be oriented differently (e.g., rotated 90 degrees or at other orientations) and the spatially relative terms used herein may be interpreted accordingly.

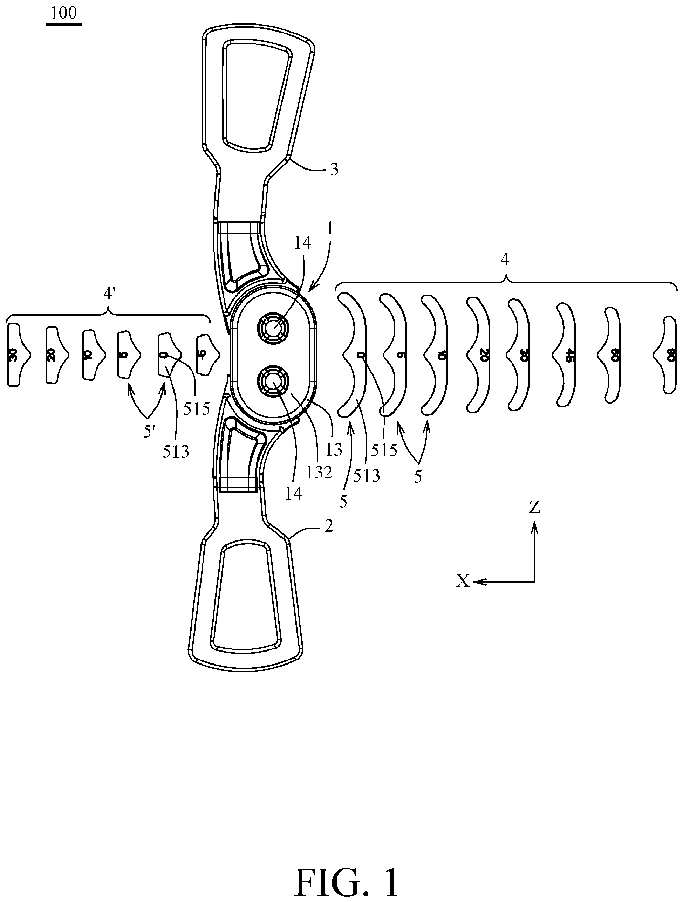

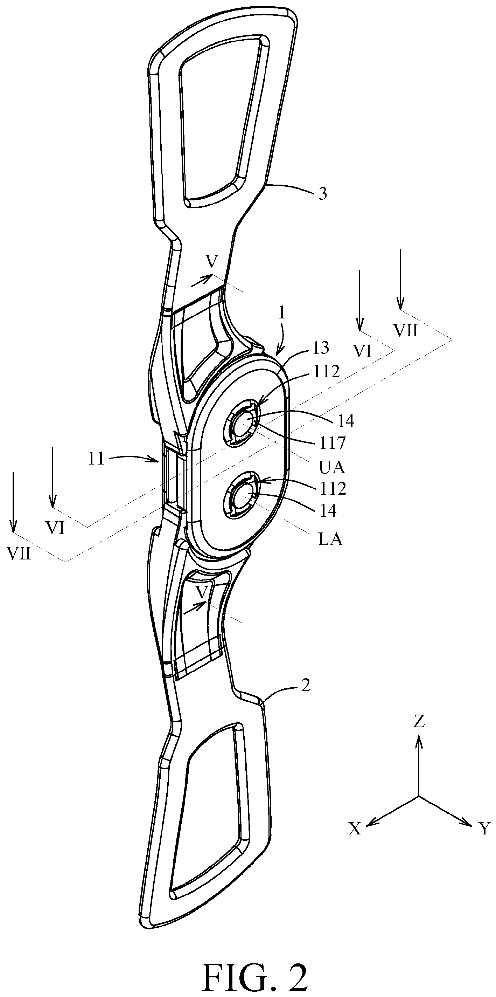

Referring to FIGS. 1 and 2, an embodiment of a hinge 100 according to the disclosure is a multi-centric hinge adapted for an orthosis (not shown), such as an orthopedic knee brace to be mounted on a leg of a user and cover the knee of the leg. The hinge 100 is for supporting the knee and for controlling and restraining the range of flexion and extension of the knee. The hinge 100 includes a housing 1, a lower strut 2, an upper strut 3, a rear angularly restraining assembly 4 and a front angularly restraining assembly 4'.

In the following description, the hinge 100 defines a front-rear direction (X), a left-right direction (Y) transverse to the front-rear direction (X), and an up-down direction (Z) transverse to both of the front-rear direction (X) and the left-right direction (Y).

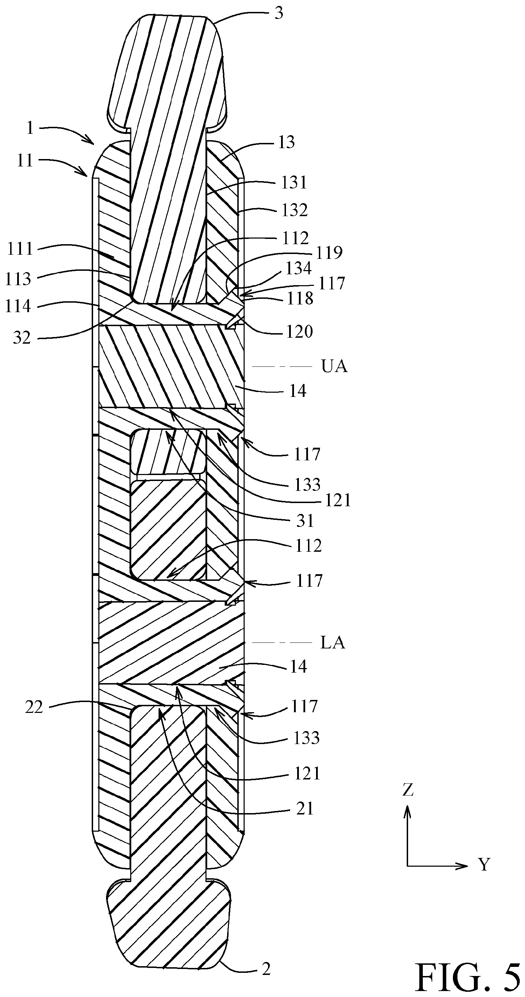

With reference to FIGS. 2, 3, 4 and 5, the housing 1 includes a housing shell 11, a cover plate 13 and two axial plugs 14. The housing shell 11 is made of a plastic material, and has an upright lateral plate 111 and two shafts 112 disposed on an inside surface 113 of the upright lateral plate 111. The upright lateral plate 111 has an outside surface 114 that is opposite to the inside surface 113 in the left-right direction (Y) and that faces a leg of a user. The shafts 112 are aligned with and spaced apart from each other in the up-down direction (Z) to respectively define the upper axis (UA) and the lower axis (LA). The upper strut 3 and the lower strut 2 are respectively hinged on the shafts 112 to be rotatable relative to the housing 1 about the upper axis (UA) and the lower axis (LA), respectively. Each shaft 112 has a tubular portion 115 integrally formed with and extending from the inside surface 113 along the lower or upper axis, and a plurality of dog portions 117 formed on a remote end of the tubular portion 115 from the inside surface 113 and surrounding and angularly spaced apart from each other about the lower or upper axis. Each dog portion 117 has a guided end surface 118 distal from the upright lateral plate 111, an engaged surface 119 extending from the guided end surface 118 toward the upright lateral plate 111, and an inner arcuate surface 120 extending from the guided end surface 118 and radially opposite to the engaged surface 119. The housing shell 11 has two axial holes 121 each of which extends through the upright lateral plate 111 and the respective shaft 112. The inner arcuate surfaces 120 of the dog portions 117 face and extend around the corresponding axial hole 121.

The cover plate 13 is made of a plastic material, and is spaced apart from the upright lateral plate 111 of the housing shell 11 in the left-right direction (Y). The cover plate 13 has an inner plate surface 131, an outer plate surface 132 opposite to the inner plate surface 131, and two engaging holes 133 which are aligned with and spaced apart from each other in the up-down direction (Z) to be respectively aligned with the shafts 112 such that the dog portions 117 of each shaft 112 extend through the respective engaging hole 133 and are retained on the cover plate 13. The cover plate 13 further has two outer annular guiding surfaces 134 which respectively surround the engaging holes 133 and extend to the outer plate surface 132 to be engaged with the engaged surfaces 119 of the dog portions 117.

Each axial plug 14 is made of a plastic material and is inserted into and seals the corresponding axial hole 121.

Each of the lower strut 2 and the upper strut 3 is made of a plastic material. The lower strut 2 and the upper strut 3 respectively have pivot holes 21, 31 aligned and in communication with the engaging holes 133, respectively, to be respectively and rotatably pivoted to the shafts 112 such that the lower strut 2 and the upper strut 3 are hinged with the housing shell 11 to be rotatable relative to the housing shell 11 about the lower axis (LA) and the upper axis (UA). The lower strut 2 and the upper strut 3 respectively have inner annular guiding surfaces 22, 32 respectively surrounding the pivot holes 21, 31 and facing the inside surface 113 of the upright lateral plate 111, and gear portions 23, 33 formed at terminal ends thereof to mesh with each other to be synchronously rotated in opposite rotational directions relative to the housing 1.

Specifically, the guided end surface 118 of each dog portion 117 is configured to be in slidable contact with the corresponding inner annular guiding surfaces 22, 32 when each dog portion 117 extends through the corresponding pivot hole 21, 31 and the corresponding engaging hole 133, which resiliently deforms the dog portion 117 to permit insertion of the dog portion 117 into the corresponding pivot hole 21, 31, where the engaged surface 119 of each dog portion 117 is engaged with the corresponding outer annular guiding surface 134. Furthermore, the engaged surface 119 is configured to be in slidable contact with the outer annular guiding surface 134 to resiliently deform the dog portion 117 for permitting removal of the dog portion 117 from the corresponding engaging hole 133. The inner arcuate surface 120 of each dog portion 117 is configured for permitting slidable contact of the corresponding axial plug 14 therewith to guide insertion of the axial plug 14 into the corresponding axial hole 121.

To assemble the housing 1 with the lower strut 2 and the upper strut 3, the cover plate 13 is abutted against the lower strut 2 and the upper strut 3 to be respectively aligned and communicated with the engaging holes 133 via the pivot holes 21, 31. The shafts 112 are then aligned with the pivot holes 21, 31 and are disposed opposite to the cover plate 13. Subsequently, the housing shell 11 is moved toward the lower strut 2 and the upper strut 3. During the movement of the housing shell 11, through the sliding contact of the guided end surfaces 118 of each shaft 112 with the corresponding inner annular guiding surface 22, 32, the dog portions 117 are resiliently deformed to permit insertion of the dog portions 117 into the corresponding pivot hole 21, 31. Then, the dog portions 117 pass through the pivot hole 21, 31 and are inserted into the corresponding engaging hole 133 until the inside surface 113 of the upright lateral plate 111 abuts against the lower strut 2 and the upper strut 3. At this stage, the engaged surfaces 119 of the dog portions 117 face and are retainingly engaged with the corresponding outer annular guiding surface 134 by the returning biasing force stored in the dog portions 117 so as to retain the dog portions 117 automatically to the cover plate 13.

Next, each axial plug 14 is inserted into the corresponding axial hole 121 from an outer side of the cover plate 13. During the insertion of each axial plug 14, once the axial plug 14 is deflected relative to a plane defined by the front-rear direction (X) and the up-down direction (Z) and contacts the inner arcuate surfaces 120 of the dog portions 117, the axial plug 14 is slidable along the inner arcuate surfaces 120 and guided thereby to guide the insertion of the axial plug 14 into the corresponding axial hole 121. Hence, the deflection of the axial plug 14 may be eliminated and the axial plugs 14 are conveniently and precisely inserted into the axial holes 121. With the axial plugs 14 being inserted into and sealing the axial holes 121, the axial plugs 14 and the upright lateral plate 111 are fittingly interfered with the shafts 112, and the housing 1 is assembled with the lower strut 2 and the upper strut 3.

When it is desired to remove the housing 1 from the lower strut 2 and the upper strut 3, a pressing force needs to be applied to each axial plug 14 to remove the axial plug 14 from the axial hole 121. The upright lateral plate 111 of the housing shell 11 is then pulled away from the cover plate 13. During the pulling movement of the upright lateral plate 111, through sliding of the engaged surfaces 119 of the dog portions 117 of each shaft 112 along the outer annular guiding surfaces 134, the dog portions 117 are resiliently deformed to permit removal of the dog portions 117 from the corresponding engaging hole 133. The dog portions 117 are then removed from the corresponding pivot hole 21, 31.

Therefore, the housing 1 is assembled with and disassembled from the lower strut 2 and the upper strut 3 without any tools so as to render assembling and disassembling operations convenient and time-saving. Moreover, with each axial plug 14 inserted into and sealing the corresponding axial hole 121, the dog portions 117 of the shaft 112 may be firmly retained to the cover plate 13 to prevent undesired removal of the dog portions 117 from the outer annular guiding surface 134 due to incorrect operation of the dog portions 117. Thus, the housing 1 is firmly assembled with the lower strut 2 and the upper strut 3.

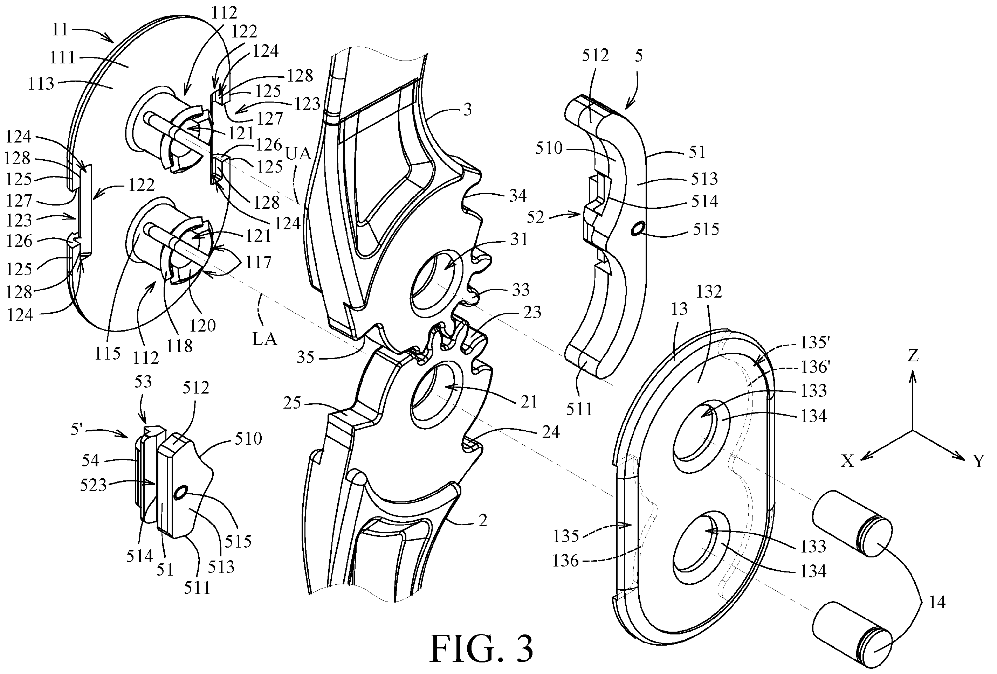

With reference to FIGS. 3, 4, 6 and 7, the housing shell 11 has two retaining slots 122 and two accommodation slots 123. The retaining slots 122 are formed in the inside surface 113 of the upright lateral plate 111 and opposite to each other in the front-rear direction (X) and recessed toward the outside surface 114. The accommodation slots 123 are formed opposite to each other in the front-rear direction (X) and extend between the inside surface 113 and the outside surface 114 to be respectively in communication with the retaining slots 122. Each retaining slot 122 has two retaining sections 124 respectively in communication with upper and lower ends of the corresponding accommodation slot 123. The upright lateral plate 111 further has two pairs of rim portions 125 which are connected with the inside surface 113. The rim portions 125 of each pair are formed at the upper and lower ends of the corresponding accommodation slot 123 and adjacent to and spaced apart from the corresponding retaining slot 122. Each accommodation slot 123 extends in the up-down direction (Z) to terminate at a lower abutting wall 126 of one rim portion 125 and an upper abutting wall 127 of the other rim portion 125. Each rim portion 125 further has an outer abutting wall 128 facing the corresponding retaining section 124.

The cover plate 13 has two inner abutting walls 136, 136' which face in the front-rear direction (X) and respectively define two positioning slots 135, 135' in the cover plate 13. Specifically, the positioning slots 135, 135' are recessed from the inner plate surface 131 toward the outer plate surface 132 and are bordered by the inner abutting walls 136, 136'.

Each of the lower strut 2 and the upper strut 3 further has a rear abutting surface 24, 34 which is formed rearwardly of the gear portion 23, 33, and a front abutting surface 25, 35 which is formed forwardly of the gear portion 23, 33.

With reference to FIGS. 1 and 3, the rear angularly restraining assembly 4 and the front angularly restraining assembly 4' are disposed opposite to each other in the front-rear direction (X). The rear angularly restraining assembly 4 includes a plurality of angularly restraining members 5 which are in the form of flexion restraining members 5 and are configured to respectively have different restraining angles such that a selected one of the angularly restraining members 5 is mounted on the housing 1 and releasably engaged in the corresponding retaining slot 122. The front angularly restraining assembly 4' includes a plurality of angularly restraining members 5' which are in the form of extension restraining members 5' and are configured to respectively have different restraining angles such that a selected one of the angularly restraining members 5' is mounted on the housing 1 and releasably engaged in the corresponding retaining slot 122.

In the embodiment, the rear angularly restraining assembly 4 includes eight angularly restraining members 5 configured to respectively have the restraining angles of 0, 5, 10, 20, 30, 45, 60 and 90 degree(s). The restraining angle of each angularly restraining member 5 is a flexion angle at which the hinge 100 may provide. Thus, a selected one of the angularly restraining members 5 according to the required flexion angle of the hinge 100 is mounted on the housing 1. The front angularly restraining assembly 4' includes six angularly restraining members 5' configured to respectively have the restraining angles of -5, 0, 5, 10, 20 and 30 degree(s). The restraining angle of each angularly restraining member 5' is an extension angle at which the hinge 100 may provide. Thus, a selected one of the angularly restraining members 5' according to the required extension angle of the hinge 100 is mounted on the housing 1.

The angularly restraining members 5, 5' with the restraining angle of 0 degree are described in the following as an example.

With reference to FIGS. 3, 4, 6 and 7, each of the angularly restraining members 5, 5' is removably mounted on the housing 1. Each of the angularly restraining members 5, 5' is made of a plastic material and integrally formed by injection molding to be a single piece. Each angularly restraining member 5, 5' includes an angularly restraining body 51, a resilient arm 52, a retaining block 53 and a removal operating block 54. The angularly restraining body 51 is disposed between and interconnects the lower strut 2 and the upper strut 3 to abut against each of the lower strut 2 and the upper strut 3 to define and limit turnable angles of the lower strut 2 and the upper strut 3. The resilient arm 52 is connected with the angularly restraining body 51 at a proximal side thereof. The retaining block 53 projects from a distal side of the resilient arm 52 that is opposite to the proximal side. The retaining block 53 is removably engaged in the corresponding retaining slot 122 to retain the angularly restraining member 5, 5' to the housing 1. The retaining block 53 is operable to bring the resilient arm 52 in resilient deformation to permit removal from the retaining slot 122. The removal operating block 54 projects from the distal side of the resilient arm 52 and is exposed from the housing 1 such that the removal operating block 54 is operably pressed to move the retaining block 53 relative to the retaining slot 122 so as to permit removal of the retaining block 53 from the retaining slot 122.

Specifically, the angularly restraining body 51 of each angularly restraining member 5 has a shape that matches those of the positioning slot 135 and the inner abutting wall 136, and the angularly restraining body 51 of each angularly restraining member 5' has a shape that matches those of the positioning slot 135' and the inner abutting wall 136' so as to permit accommodation of the angularly restraining bodies 51 of the angularly restraining members 5, 5' in the positioning slots 135, 135', respectively. With the angularly restraining bodies 51 of a variety of shapes mounted in the housing 1, the angularly restraining members 5, 5' may define a variety of restraining angles. The angularly restraining body 51 has a fitting surface 510 extending in the up-down direction (Z), a lower abutted surface 511 connected with a lower end of the fitting surface 510, and an upper abutted surface 512 connected with an upper end of the fitting surface 510. The fitting surface 510 is configured to fit with and abut against the corresponding inner abutting wall 136, 136'. The lower abutted surface 511 and the upper abutted surface 512 of the angularly restraining member 5 are disposed to respectively abut against the rear abutting surface 24 of the lower strut 2 and the rear abutting surface 34 of the upper strut 3 to define and limit the rotating angle of the lower strut 2 and the upper strut 3. The lower abutted surface 511 and the upper abutted surface 512 of the angularly restraining member 5' are disposed to respectively abut against the front abutting surface 25 of the lower strut 2 and the front abutting surface 35 of the upper strut 3 to define and limit the rotating angle of the lower strut 2 and the upper strut 3. The angularly restraining body 51 further has an indicating upright surface 513 facing the cover plate 13, and a connecting upright surface 514 opposite to the indicating upright surface 513 in the left-right direction (Y) and facing the upright lateral plate 111. The upright indicating surface 513 is marked with a number 515 indicating a restraining angle.

The resilient arm 52 has a first arm portion 521 which extends from the connecting upright surface 514 away from the indicating upright surface 513, and a second arm portion 522 which extends transversely from a terminal end of the first arm portion 521 and which faces and is spaced apart from the upright connecting surface 514 with a gap 523.

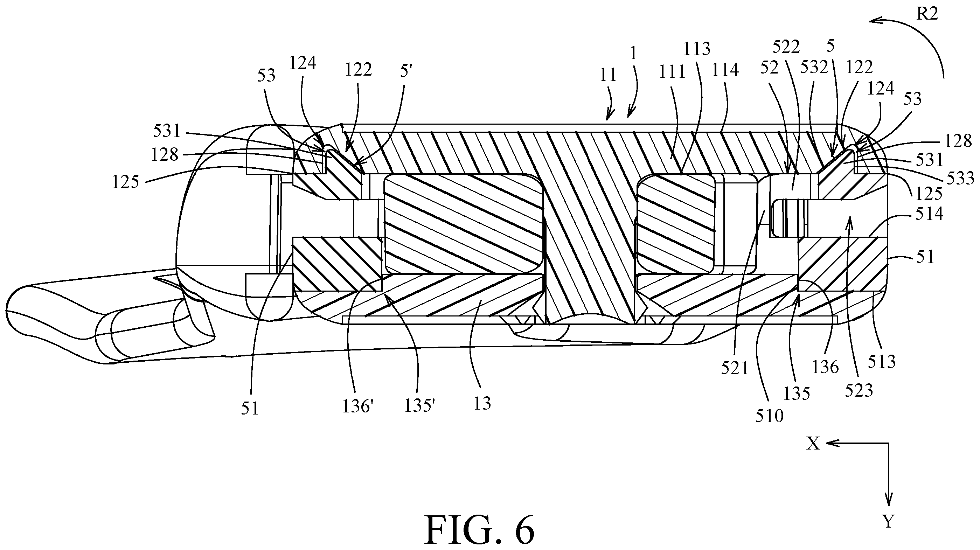

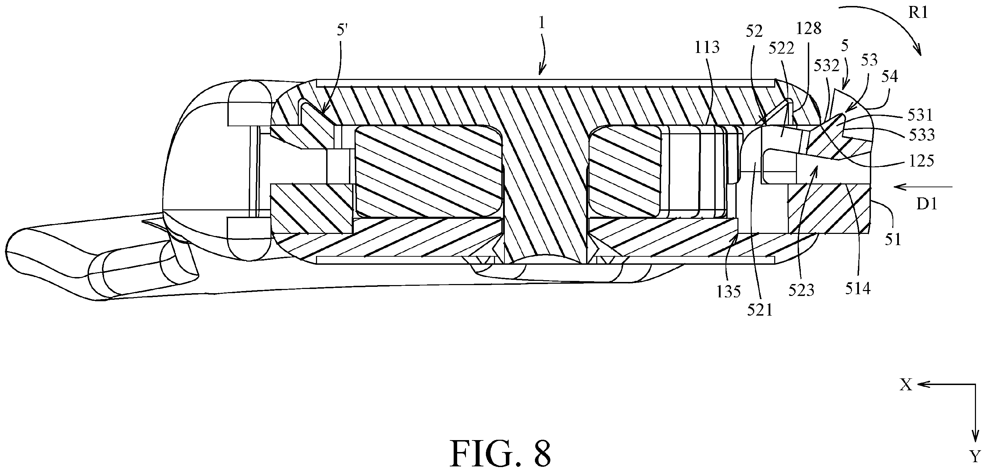

The retaining block 53 projects from the second arm portion 522 away from the angularly restraining body 51, and has two retaining protrusions 531 opposite to each other in the up-down direction (Z) to be respectively and retainingly engaged in the retaining sections 124 of the corresponding retaining slot 122. Each retaining protrusion 531 has a slope surface 532 formed proximal to the first arm portion 521, and an engaging surface 533 formed opposite to the slope surface 532 and distal from the first arm portion 521. The slope surface 532 is in slidable contact with the rim portion 125 to bring the resilient arm 52 in resilient deformation (see FIG. 8) so as to permit engagement of the retaining protrusion 531 in the corresponding retaining section 124. The engaging surface 533 is formed to be retainingly engaged in the retaining slot 122 when the resilient arm 52 is returned to an original form thereof and in abutting engagement with the corresponding outer abutting wall 128 (see FIG. 6).

The removal operating block 54 projects from the second arm portion 522 away from the angularly retaining body 51, and is connected between the two retaining protrusions 531. The removal operating block 54 is accommodated in the corresponding accommodation slot 123 such that the removal operating block 54 has an operating portion exposed from the housing 1 through the accommodation slot 123 for manual operation by the user.

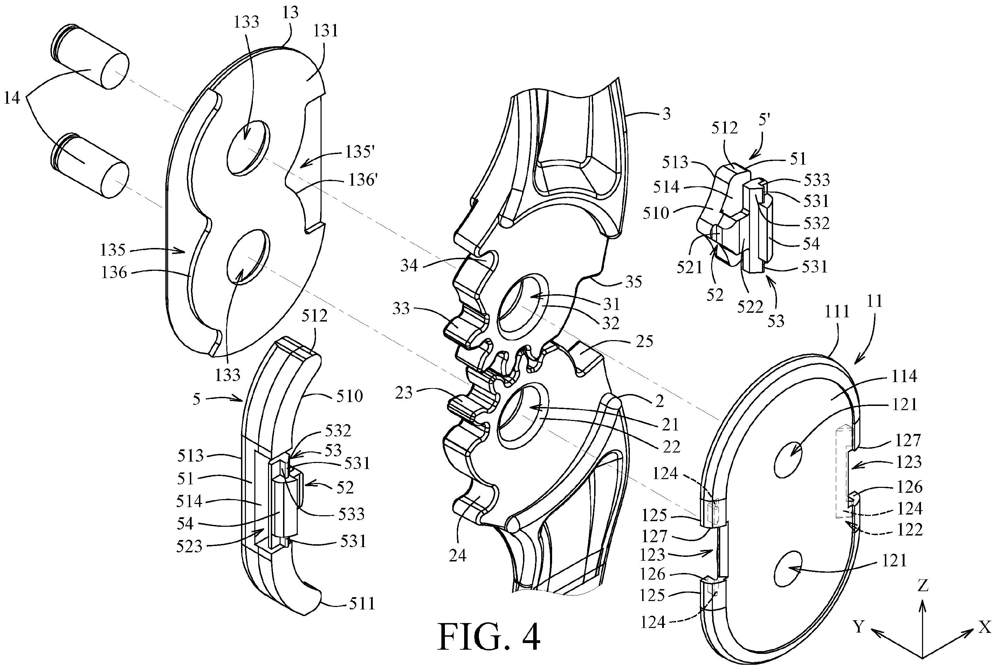

With reference to FIGS. 4 and 8, to assemble the angularly restraining member 5 with the housing 1, the angularly restraining body 51 and the removal operating block 54 are respectively aligned with the positioning slot 135 and the corresponding accommodation slot 123, and the angularly restraining member 5 is moved along an assembled direction (D1) parallel to the front-rear direction (X) to insert the angularly restraining body 51 into the positioning slot 135. Subsequently, through the sliding contact of the slope surface 532 of each retaining protrusion 531 with the corresponding rim portion 125, the second arm portion 522 of the resilient arm 52 is resiliently deformed toward the gap 523 to permit slight rotation of the retaining block 53 and the removal operating block 54 in a first rotational direction (R1). Thus, each retaining protrusion 531 of the retaining block 53 is moved over the corresponding rim portion 125 and along the inside surface 113 of the upright lateral plate 111.

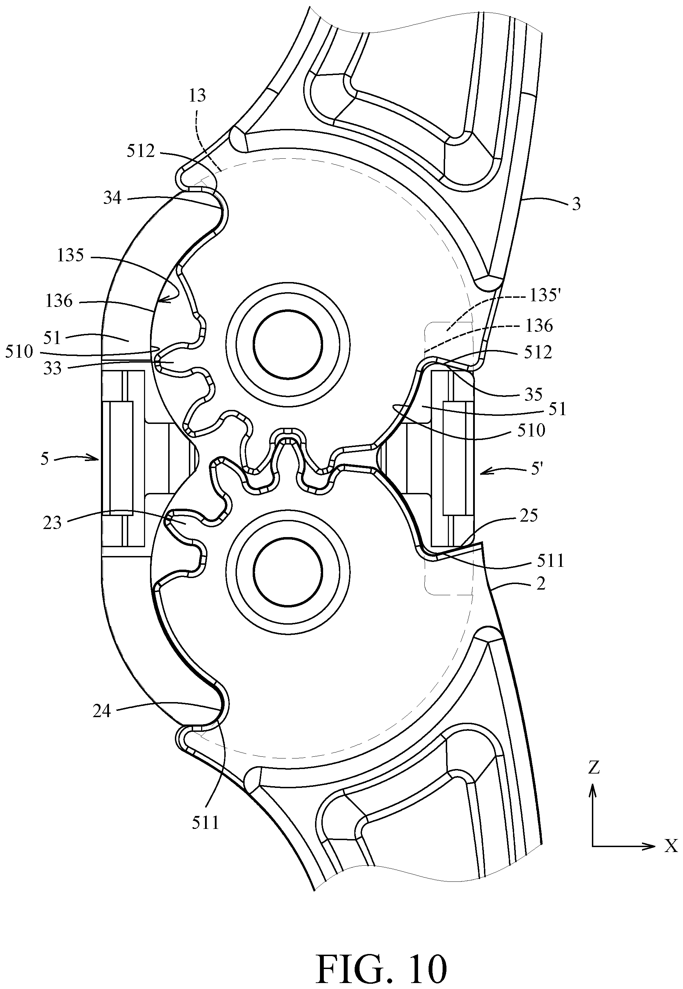

With reference to FIGS. 6, 9 and 10, when the fitting surface 510 of the angularly restraining body 51 of the angularly restraining member 5 abuts against the inner abutting wall 136, the forward movement of the angularly restraining member 5 is stopped. Thus, the angularly restraining body 51 is prevented from collision with the gear portions 23, 33 of the lower strut 2 and the upper strut 3 and from interference with rotation of the gear portions 23, 33. At the same time, the retaining protrusions 531 of the retaining block 53 are respectively aligned with the retaining sections 124. By the returning biasing force stored in the second arm portion 522 of the resilient arm 52, the retaining block 53 and the removal operating block 54 are rotated in a second rotational direction (R2) opposite to the first rotational direction (R1) (see FIG. 8) and returned to their original shape so as to bring the retaining protrusions 531 automatically and retainingly engaged in the retaining sections 124 and the removal operating block 54 automatically retained in the corresponding accommodation slot 123.

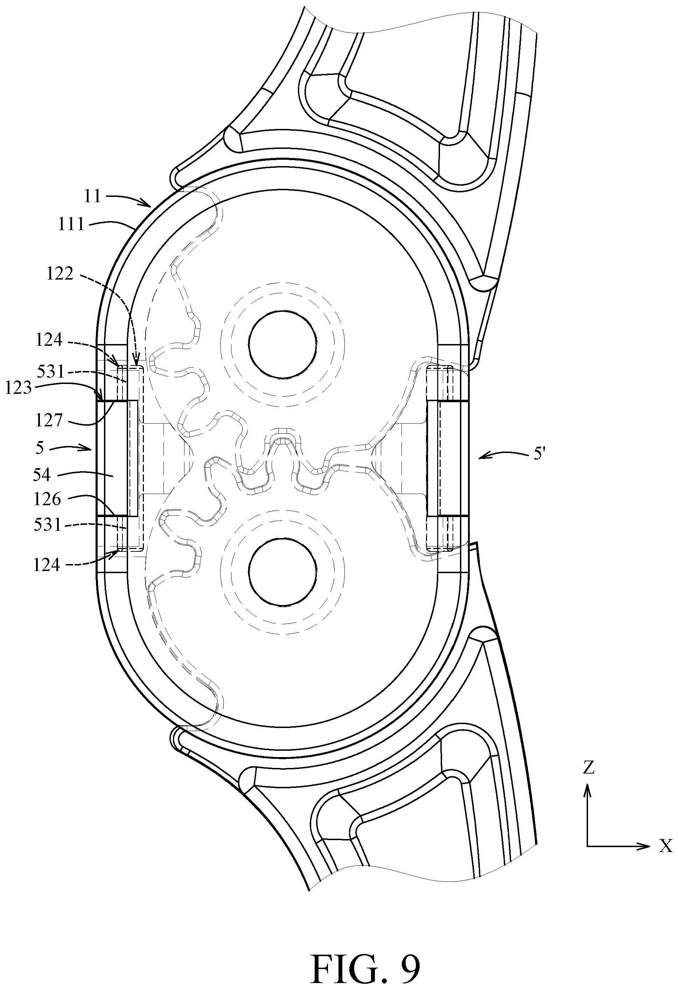

With the inner abutting wall 136 and the corresponding outer abutting wall 128 respectively in abutting engagement with the fitting surface 510 of the angularly restraining body 51 and the engaging surface 533 of the corresponding 531, swing movement of the angularly restraining member 5 in the front-rear direction (X) is prevented. With the fitting surface 510 in abutting engagement with the inner abutting wall 136, and with the upper abutting wall 127 and the lower abutting wall 126 in abutting engagement with the removal operating block 54 (see FIG. 9), the swing movement of the angularly restraining member 5 in the up-down direction (Z) is prevented. With the cover plate 13 and the upright lateral plate 111 respectively in abutting engagement with the angularly restraining body 51 and both the resilient arm 52 and the retaining block 53, the swing movement of the angularly restraining member 5 in the left-right direction (Y) is prevented. Furthermore, with the two retaining sections 124 of the retaining slot 122, and with the two retaining protrusions 531 of the retaining block 53 projecting from the upper and lower ends of the removal operating block 54 to be engaged in the retaining sections 124, the retaining strength of the angularly restraining member 5 to the housing 1 is enhanced such that the angularly restraining member 5 is firmly retained to the housing 1.

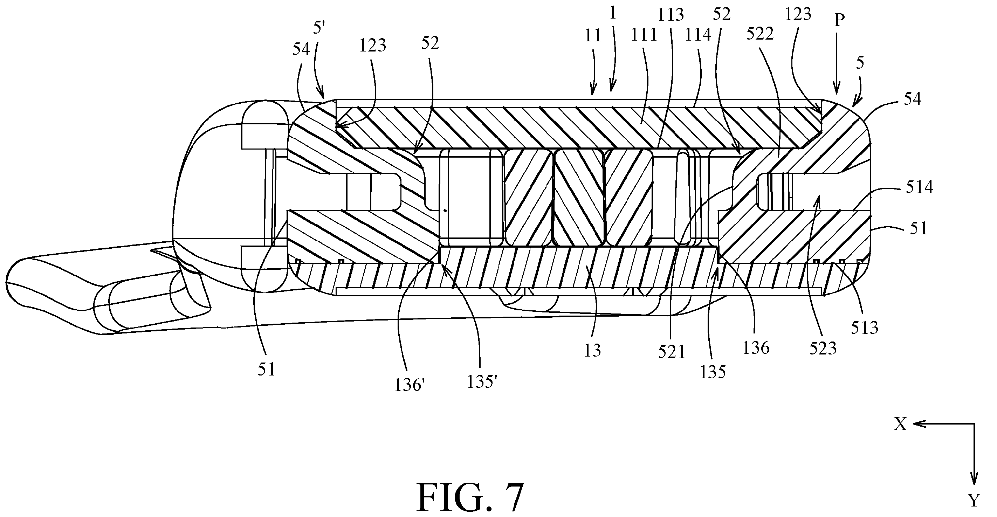

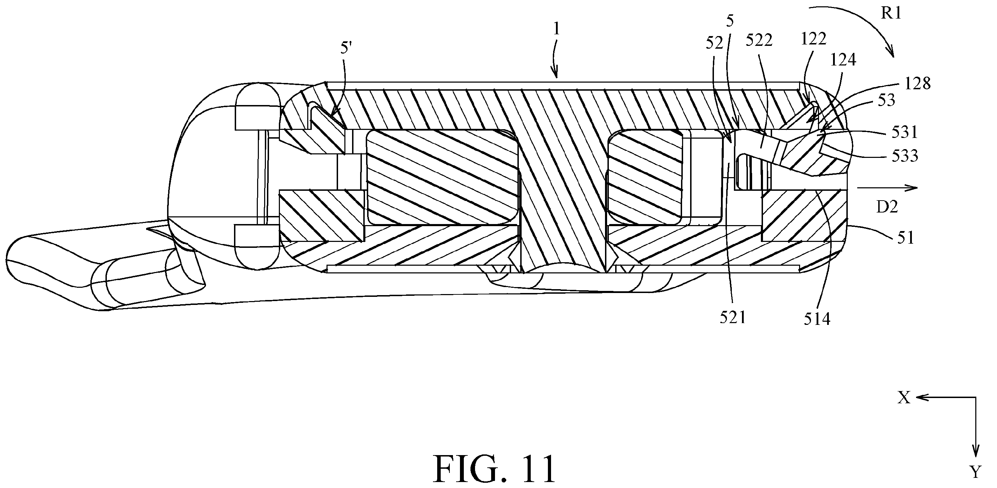

With reference to FIGS. 7 and 11, when it is desired to remove the angularly restraining member 5 from the housing 1 for replacing another angularly restraining member 5 with a different restraining angle, the removal operating block 54 is pressed in a pressing direction (P) parallel to the left-right direction (Y) to resiliently deform the second arm portion 522 of the resilient arm 52 relative to the first arm portion 521 toward the upright connecting surface 514, and rotate the retaining block 53 in the first rotational direction (R1) such that the retaining protrusions 531 are disengaged from the retaining sections 124. Subsequently, the angularly restraining member 5 is pulled rearwardly in a disassembled direction (D2) opposite to the assembled direction (D1) (see FIG. 8) to be removed from the housing 1.

With reference to FIGS. 6 and 8, with the slope surface 532 of each retaining protrusion 531 of the retaining block 53, the angularly restraining member 5 may be assembled with and retained to the housing 1 by inserting the angularly restraining member 5 into the housing 1 along the assembled direction (D1). Thus, the assembling operation of the angularly restraining member 5 is convenient and easy to conduct.

With reference to FIGS. 7 and 11, with the structural connection among the resilient arm 52, the retaining block 53 and the removal operating block 54, the retaining of the retaining block 53 may be released by pressing the removal operating block 54 along the pressing direction (P). Subsequently, the angularly restraining member 5 may be removed from the housing 1 by pulling the angularly restraining member 5 along the disassembled direction (D2). Thus, the disassembling operation of the angularly restraining member 5 is convenient and easy to conduct.

It is noted that, since the angularly restraining member 5' is assembled with and disassembled from the housing 1 in a similar manner to that of the angularly restraining member 5, a description thereof is omitted.

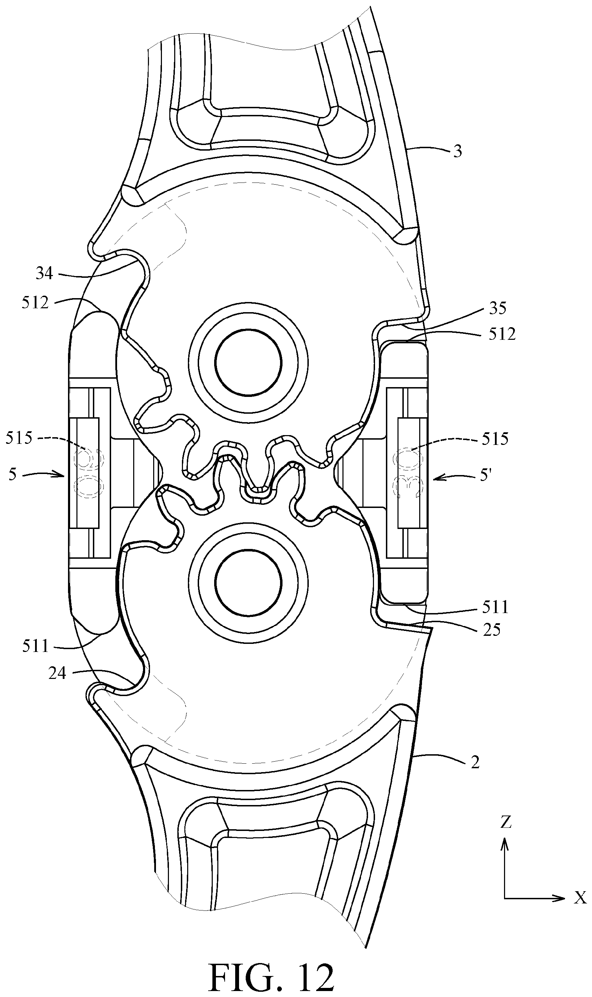

With reference to FIG. 12, which illustrates a state in which the angularly restraining members 5, 5' with different restraining angles are being assembled with the housing 1. In this state, the restraining angle of the angularly restraining member 5 is 90 degrees, and the restraining angle of the angularly restraining member 5' is 30 degrees.

With reference to FIGS. 4 and 6, with each of the angularly restraining members 5, 5' being integrally formed in a single piece and having the retaining block 53 and the removal operating block 54, each angularly restraining member 5, 5' has both of the engaged and disengaged operating functions. Hence, by virtue of the assembling operation of the angularly restraining member 5, 5' with the housing 1 and by virtue of the disassembling operation of the angularly restraining member 5, 5' from the housing 1, the flexion angle and the extension angle of the hinge 100 (as shown in FIG. 1) may be adjusted conveniently and easily so as to perform an efficient adjustment of the rotational angle of the hinge 100. Moreover, the assembling and disassembling operations are performed without any tools such that the number of components of the hinge 100 is reduced to render the structure of the hinge 100 simple and the manufacturing cost thereof reduced.

With reference to FIG. 1, the upright indicating surface 513 of each angularly restraining member 5, 5' is marked with a number 515 indicating a restraining angle. The user may easily see the indicated number 515 to select a required angularly restraining member 5, 5', and correctly mount the selected angularly restraining member 5, 5' on the housing 1 by bringing the indicated number 515 on an outside of the housing 1, thereby making the operation foolproof.

Alternatively, the hinge 100 may be embodied in a modified form as follows.

In one modified embodiment, the number of the retaining slot 122 and the accommodation slot 123 of the housing 1 may be one, and the hinge 100 only includes the rear angularly restraining assembly 4.

In one modified embodiment, the number of the retaining slot 122 and the accommodation slot 123 of the housing 1 may be one, and the hinge 100 only includes the front angularly restraining assembly 4'.

In one modified embodiment, the number of the retaining section 124 of the retaining slot 122, and the number of the rim portion 125 corresponding to the retaining section 124 may be one. The number of the retaining protrusion 531 of the retaining block 53 may be one.

In one modified embodiment, the number of the angularly restraining member 5 of the rear angularly restraining assembly 4 and the number of the angularly restraining member 5' of the front angularly restraining assembly 4' may be one.

## Claims

1. A hinge (100), comprising:
a housing (1) having at least one retaining slot (122) formed therein;
a lower strut (2) hinged with said housing (1) to be rotatable relative to said housing (1) about a lower axis (LA); and
an upper strut (3) hinged with said housing (1) to be rotatable relative to said housing (1) about an upper axis (UA), said upper strut (3) being connected with said lower strut (2) to be synchronously rotated with said lower strut (2) in opposite rotational directions, **characterized by**:
at least one angularly restraining member (5, 5') removably mounted on said housing (1), and including an angularly restraining body (51) which is disposed between and interconnects said lower strut (2) and said upper strut (3) to abut against each of said lower strut (2) and said upper strut (3) to define turnable angles of said lower strut (2) and said upper strut (3), a resilient arm (52) which is connected with said angularly restraining body (51) at a proximal side thereof, and a retaining block (53) which projects from a distal side of said resilient arm (52) that is opposite to said proximal side and which is removably engaged in said retaining slot (122) to retain said angularly restraining member (5, 5') to said housing (1), said retaining block (53) being operable to bring said resilient arm (52) in resilient deformation to permit removal from said retaining slot (122).

2. The hinge as claimed in claim 1, wherein said housing (1) has at least one rim portion (125) which is formed adjacent to and spaced apart from said retaining slot (122), said retaining block (53) having a slope surface (532) which is in slidable contact with said rim portion (125) to bring said resilient arm (52) in resilient deformation, and an engaging surface (533) which is opposite to said slope surface (532) and retainingly engaged in said retaining slot (122) when said resilient arm (52) is returned to an original form thereof.

3. The hinge as claimed in claim 1, wherein said angularly restraining member (5, 5') further includes a removal operating block (54) which projects from said distal side of said resilient arm (52) and is exposed from said housing (1) such that said removal operating block (54) is operably pressed to move said retaining block (53) relative to said retaining slot (122) so as to permit removal of said retaining block (53) from said retaining slot (122).

4. The hinge as claimed in claim 3, wherein said housing (1) further has at least one accommodation slot (123) which is formed therethrough and in communication with said retaining slot (122) for accommodating said removal operating block (54), and at least one rim portion (125) which is formed at an end of said accommodation slot (123), said removal operating block (54) having an operating portion exposed from said housing (1) through said accommodation slot (123), said retaining slot (122) having at least one retaining section (124) in communication with said end of said accommodation slot (123) and is spaced apart from said rim portion (125), said retaining block (53) having at least one retaining protrusion (531) which is connected with an end of said removal operating block (54), said retaining protrusion (531) having a slope surface (532) which is in slidable contact with said rim portion (125) to bring said resilient arm (52) in resilient deformation, and an engaging surface (533) which is opposite to said slope surface (532) and retainingly engaged in said retaining section (124) when said resilient arm (52) is returned to an original form thereof.

5. The hinge as claimed in claim 4, wherein said retaining slot (122) has two of said retaining sections (124) which are respectively in communication with an upper end and a lower end of said accommodation slot (123), said housing (1) having two of said rim portions (125) which are respectively formed at said upper end and said lower end of said accommodation slot (123) and are respectively spaced apart from said retaining sections (124), said retaining block (53) having two of said retaining protrusions (531) which are respectively connected with an upper end and a lower end of said removal operating block (54) such that said retaining protrusions (531) are respectively and retainingly engaged in said retaining sections (124).

6. The hinge as claimed in claim 1, wherein said housing (1) has at least one inner abutting wall (136, 136') which faces in a front-rear direction (X) and defines a positioning slot (135, 135') in said housing (1) for accommodating said angularly restraining body (51), and at least one outer abutting wall (128) which faces said positioning slot (135, 135') and is spaced apart from said inner abutting wall (136, 136') in the front-rear direction (X) such that said inner abutting wall (136, 136') and said outer abutting wall (128) are in abutting engagement with said angularly restraining body (51) and said retaining block (53), respectively.

7. The hinge as claimed in claim 6, wherein said housing (1) further has at least one accommodation slot (123) which is formed therethrough and in communication with said retaining slot (122), said accommodation slot (123) extending in an up-down direction (Z) to terminate at a lower abutting wall (126) and an upper abutting wall (127), said angularly restraining member (5, 5') further including a removal operating block (54) which projects from said distal side of said resilient arm (52) and is accommodated in said accommodation slot (123), said lower abutting wall (126) and said upper abutting wall (127) being in abutting engagement with said removal operating block (54) in the up-down direction (Z).

8. The hinge as claimed in claim 1, wherein said angularly restraining body (51) has an indicating upright surface (513) and a connecting upright surface (514) opposite to said indicating upright surface (513), said resilient arm (52) having a first arm portion (521) which extends from said connecting upright surface (514) away from said indicating upright surface (513), and a second arm portion (522) which extends transversely from a terminal end of said first arm portion (521) and which faces and is spaced apart from said upright connecting surface (514) with a gap (523) such that said second arm portion (522) is deformable and bent toward said upright connecting surface (514), said retaining block (53) projecting from said second arm portion (522) away from said angularly restraining body (51), said angularly restraining member (5, 5') further including a removal operating block (54) which projects from said second arm portion (522) away from said angularly restraining body (51) and is exposed from said housing (1).

9. The hinge as claimed in claim 1, wherein said housing (1) includes a housing shell (11) and a cover plate (13), said housing shell (11) having an upright lateral plate (111) and two shafts (112) which are disposed on an inside surface (113) of said upright lateral plate (111), said shafts (112) being spaced apart from each other in an up-down direction (Z) to respectively define said upper axis (UA) and said lower axis (LA) such that said upper strut (3) and said lower strut (2) are hinged on said shafts (112), respectively, each of said shafts (112) having a plurality of dog portions (117) surrounding and angularly spaced apart from each other about a respective one of said lower axis (LA) and said upper axis (UA), said cover plate (13) having two engaging holes (133) which are respectively aligned with said shafts (112) such that said dog portions (117) of each of said shafts (112) extend through a respective one of said engaging holes (133) and are retained on said cover plate (13).

10. The hinge as claimed in claim 9, wherein said lower strut (2) and said upper strut (3) respectively have pivot holes (21, 31) aligned and in communication with said engaging holes (133), respectively, to be respectively and rotatably pivoted to said shafts (112), and inner annular guiding surfaces (22, 32) respectively surrounding said pivot holes (21, 31) and facing said inside surface (113) of said upright lateral plate (111), said cover plate (13) having two outer annular guiding surfaces (134) which respectively surround said engaging holes (133), each of said dog portions (117) extending through a corresponding one of said pivot holes (21, 31) and a corresponding one of said engaging holes (133), and having a guided end surface (118) which is configured to be in slidable contact with a corresponding one of said inner annular guiding surfaces (22, 32) to resiliently deform said dog portion (117) for permitting insertion of said dog portion (117) into said pivot hole (21, 31), and an engaged surface (119) which is engaged with a corresponding one of said outer annular guiding surfaces (134) when said dog portion (117) is inserted into said pivot hole (21, 31), and which is configured to be in slidable contact with said outer annular guiding surface (134) to resiliently deform said dog portion (117) for permitting removal of said dog portion (117) from a corresponding one of said engaging holes (133).

11. The hinge as claimed in claim 9, wherein said housing shell (11) has two axial holes (121) each of which extends through said upright lateral plate (111) and a respective one of said shafts (112), and two axial plugs (14) which are inserted into and seal said axial holes (121), respectively.

12. The hinge as claimed in claim 11, wherein each of said dog portions (117) has an inner arcuate surface (120) extending around a corresponding one of said axial holes (121) and configured for permitting slidable contact of a corresponding one of said axial plugs (14) therewith to guide insertion of said axial plug (14) into said axial hole (121).

13. The hinge as claimed in claim 1, wherein said angularly restraining member (5, 5') is made of a plastic material and integrally formed to be a single piece.

14. The hinge as claimed in claim 1, wherein said housing (1) has two of said retaining slots (122) which are formed therethrough and opposite to each other in a front-rear direction (X), said hinge (100) comprising two of said angularly restraining members (5, 5') which are disposed opposite to each other in the front-rear direction (X), said angularly restraining members (5, 5') being in form of a flexion restraining member (5) and an extension restraining member (5'), respectively, and being respectively and removably engaged in said retaining slots (122).

15. The hinge as claimed in claim 1, wherein said housing (1) has two of said retaining slots (122) which are formed therethrough and opposite to each other in a front-rear direction (X), said hinge (100) comprising a rear restraining assembly (4) and a front restraining assembly (4') which are disposed opposite to each other in the front-rear direction (X), each of said rear restraining assembly (4) and said front restraining assembly (4') including a plurality of said angularly restraining members (5, 5'), wherein
said angularly restraining members (5) of said rear restraining assembly (4) are in form of flexion restraining members (5) and are configured to respectively have different restraining angles such that a selected one of said angularly restraining members (5) is mounted on said housing (1) and releasably engaged in a corresponding one of said retaining slots (122), and
said angularly restraining members (5') of said front restraining assembly (4') are in form of extension restraining members (5') and are configured to respectively have different restraining angles such that a selected one of said angularly restraining members (5') is mounted on said housing (1) and releasably engaged in a corresponding one of said retaining slots (122).
